(19) Europäisches Patentamt
European Patent Office
Office européen des brevets

(11) EP 2 316 414 B1

(12) **EUROPEAN PATENT SPECIFICATION**

(45) Date of publication and mention of the grant of the patent:
**30.03.2016 Bulletin 2016/13**

(21) Application number: **09805073.5**

(22) Date of filing: **07.08.2009**

(51) Int Cl.:
***A61K 8/34*** *(2006.01)* ***A61K 8/19*** *(2006.01)*
***A61K 8/86*** *(2006.01)* ***A61Q 5/12*** *(2006.01)*
***A61K 8/41*** *(2006.01)* ***A61K 8/42*** *(2006.01)*
***A61K 8/36*** *(2006.01)* ***A61K 8/39*** *(2006.01)*

(86) International application number:
**PCT/JP2009/064063**

(87) International publication number:
**WO 2010/016591 (11.02.2010 Gazette 2010/06)**

(54) **HAIR CONDITIONER COMPOSITION**

HAARPFLEGEZUSAMMENSETZUNG

COMPOSITION D'APRÈS-SHAMPOOING

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **08.08.2008 JP 2008206157**

(43) Date of publication of application:
**04.05.2011 Bulletin 2011/18**

(73) Proprietor: **Shiseido Company, Ltd.**
**Chuo-ku**
**Tokyo 104-8010 (JP)**

(72) Inventors:
- **KINOSHITA Kouichi**
**Yokohama-shi**
**Kanagawa 224-8558 (JP)**
- **MIYAHARA Reiji**
**Yokohama-shi**
**Kanagawa 224-8558 (JP)**
- **TESHIGAWARA Takashi**
**Yokohama-shi**
**Kanagawa 224-8558 (JP)**
- **NAGARE Yuko**
**Yokohama-shi**
**Kanagawa 224-8558 (JP)**
- **KUROKAWA Kenji**
**Yokohama-shi**
**Kanagawa 224-8558 (JP)**

(74) Representative: **Lippert, Stachow & Partner**
**Patentanwälte**
**Frankenforster Strasse 135-137**
**51427 Bergisch Gladbach (DE)**

(56) References cited:
**WO-A1-03/000205** **WO-A1-2006/119042**
**WO-A1-2010/090219** **GB-A- 2 383 950**
**JP-A- 9 165 321** **JP-A- 2001 055 309**
**JP-A- 2003 300 812** **JP-T- 2004 534 807**
**JP-T- 2005 516 026** **JP-U- 7 002 435**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 316 414 B1

## Description

RELATED APPLICATIONS

This application claims the priority of Japanese Patent Application No. 2008-206157 filed on August 8, 2008.

FIELD OF THE INVENTION

The present invention relates to a hair conditioning composition and particularly relates to a concentrated hair conditioner that is diluted when used.

BACKGROUND OF THE INVENTION

Many of conventional hair conditioners adopt a form in which components are dissolved or dispersed in a large amount of water. Products with such form are basically realized by stably dispersing various formulation components in water that is an excellent solvent. In addition, in most hair conditioners, a cationic surfactant that is a basic component forms a lamellar $\alpha$-gel structure in combination with a higher alcohol and water or via emulsification of oil and water, and the hair conditioners may turn into a high-viscosity gel. Therefore, also for viscosity control of a composition, it has been preferred to contain a large amount of water up to an extent that would not impair its usability and its texture by excessively diluting the components.

On the other hand, the product containing a large amount of water requires energy for transportation and transfer for its weight and bulkiness. In addition, water or the composition containing water has a problem in energy efficiency on the product manufacturing since energy consumption is excessively required for its heating and cooling. Furthermore, the product containing a large amount of water is susceptible to the conditions during production and storage (history of load stress and temperature) and is difficult to maintain qualities such as viscoelasticity for a long period of time.

Therefore, reduction in the water content of hair conditioner leads to reductions in a lot of energy consumption in quality maintenance, production and transportation, and in product cost, and is believed to be one of the breakthroughs in the art also from the viewpoint of improvement in global environment.

In addition, also in the standpoint of a user, a lightweight and compact hair cosmetic that can be carried on the plane where liquid and cream products are restricted and can meet also for outdoor use has been desired.

Patent Literature 1: PCT Japanese Translation Patent Publication No. 2004-534807
Patent Literature 2: PCT Japanese Translation Patent Publication No. 2005-516026
Patent Literature 3: Japanese Unexamined Patent Publication No. 2003-300812

WO 2006/119042A1 discloses a hair conditioning lamellar oil-in-glycol gel composition (a) comprising at least (a) at least one glycol, (b) at least one fatty alcohol, (c) at least one cationic surfactant and furthermore straight chain alcohols having 16 or more carbon atoms.

DISCLOSURE OF THE INVENTION

PROBLEM TO BE SOLVED BY THE INVENTION

First, as means for reducing the water content of a hair conditioning composition, application of freeze drying or spray drying widely used for foods and the like can be considered. However, in order to carry out the means, it is first required to produce a normal hair conditioner, and the energy to put the normal hair conditioner to a drying process is further added. Therefore, the energy efficiency in the production process is worse than that of a conventional hair conditioner. Also, the drying process has caused a problem such that scent of the product is impaired, or equipment investment is required for mass production. Therefore, the production of a concentrated hair conditioner in which the water content is reduced from the first has been desired.

As a hair conditioning composition with a low water content, for example, a solid hair conditioning agent is disclosed (Patent Literature 1). This is solidified (stick) form obtained by reducing the water content of a conventional hair conditioning agent and can be used by rubbing into hair.

This hair conditioning agent is the one where water of the conventional formulation components is reduced, in other words, water is used as a concentrated medium. In this case, the amount of water contained is reduced, and viscosity is markedly increased during production, and thus, stirring and mixing of the components are markedly prevented

with a reduction of the water content. Therefore, there is a limit to the amount of water that can be removed from the hair conditioning agent using water as a medium.

In addition, as another invention of a solid hair conditioning agent with a low water content, an agent in which relatively stable production is made possible by dissolving the components into oil such as warmed cocoa butter has been developed (Patent Literature 2). However, with a use mode of rubbing into hair, it is difficult to homogeneously apply the conditioner to entire use site, and also in the nature of directly rubbing solidified oil into hair, the solid hair conditioning agent falls short of a conventional water-based hair conditioning agent in an aspect of ease of handling such as stickiness or the like.

In other words, in spite of the problem of viscosity increase of the system, it is possible to reduce the water content of the hair conditioning composition to some extent and substitute water with oil, while it is difficult to concentrate conditioning components with containing almost no water. Therefore, a concentrated hair conditioner that is a type to be diluted with water before use is not yet realized as a product.

Patent Literature 3 describes a flake form cosmetic base composition containing a high concentration of a cationic surfactant with a specific structure that can be used as a conditioning component. However, while the base composition has very high hygroscopicity, the base composition has low water absorbability and is difficult to be diluted with water. Therefore, the base composition cannot be used as a concentrated hair conditioner as it is.

The present invention has been made in view of the problems, and an object of the present invention is to provide an easily-handled hair conditioning composition with a very low water content.

MEANS TO SOLVE THE PROBLEM

The present inventors have extensively investigated and consequently found that, in compositions containing a polyhydric alcohol and/or polyethylene glycol as a medium of a higher alcohol and a cationic surfactant that are conditioning components, those having a melting temperature of a gel by these components of 50°C or more and a melting temperature of the medium of 155°C or less can be used as an easily-handled hair conditioning composition, thereby accomplishing the present invention.

More specifically, the hair conditioning composition of the present invention is a composition comprising:

(a) 10 to 90% by mass of one or more components selected from

(i) higher alcohols, being straight chain alcohols having 16 or more carbon atoms, and
(ii) higher fatty acids, having 12 to 22 carbon atoms, and
(iii) derivatives thereof, represented by following formulas (I) and (II)

$$R^1\text{-O-(-(CH}_2)_y\text{-O-)}_x\text{-H} \quad \text{(I)}$$

wherein in the formula (I), $R^1$ is a straight chain or branched fatty alcohol residue having 10 to 24 carbon atoms, and each of x and y is an integer of 1 to 3,

$$R^2\text{-COO-CH}_2\text{-CH(OH)-CH}_2\text{-Q} \quad \text{(II)}$$

wherein $R^2$ is a straight chain or branched fatty acid residue having 9 to 23 carbon atoms, and Q is H or OH;

(b) 5 to 35% by mass of a cationic surfactant, and
(c) one or more components selected from the group consisting of erythritol, maltitol, sorbitol, xylitol and/or polyethylene glycol with a molecular weight of 3,000 to 5,000,000, having a melting point of 155°C or less,

wherein the endothermic peak of a gel which is formed from (a) and (b) in the composition is 50°C or more as measured by a differential scanning calorimeter (DSC),
wherein the water content is 10% by mass or less, and
wherein the molar ratio of (a) to (b) is 2.5 or more to less than 6.0.

Furthermore, the present inventors have found that specific polyhydric alcohol and/or polyethylene glycol is applied as a medium, and a component selected from higher alcohols, higher fatty acids, and derivatives thereof, and a cationic surfactant are contained in a specific molar ratio, thereby providing low hygroscopicity and high water absorbability to the hair conditioning composition.

More specifically, in the hair conditioning composition, (c) is preferably erythritol, maltitol and/or polyethylene glycol with a molecular weight of 3,000 to 300,000.

In the hair conditioning composition, the molar ratio of (a) to (b) is 2.5 or more to less than 6.0.

Further, in the hair conditioning composition, the cationic surfactant is preferably a mono long-chain alkyl type.
Furthermore, the hair conditioning composition of the present invention is preferably a solid or powder form at ambient temperature.
In addition, the hair conditioning composition is preferably a hair conditioning precursor composition.
Also, the hair conditioning composition is preferably diluted with water at a dilution rate of 3 to 15 times by mass before use.
Further, the method for producing a hair conditioner of the present invention is characterized by mixing a hair conditioning composition with water.
Moreover, the method of using a hair conditioning composition of the present invention is characterized by mixing the composition with water.

EFFECT OF THE INVENTION

According to the present invention, a concentrated type hair conditioning composition that can be diluted with water before its application to hair can be obtained. Since the composition can reduce energy consumption during production and transportation and reduce energy consumption required for use and disposal of a container and an outer package without degrading the quality as a hair conditioner, contribution to the improvement in global environment can be expected. In addition, the hair conditioning composition of the present invention contains very little water and is thus compact, and can be used in an easily-handled form, thereby having a great advantage in carrying on the plane and for outdoor use.

BEST MODE FOR CARRYING OUT THE INVENTION

When a hair conditioning composition of the type which is diluted with water before use is produced, a mode in which the water of a conventional hair conditioning composition containing a large amount of water is reduced, and this water is compensated when used can be first considered. However, when a general hair conditioning composition is produced with low water content, in a gel ($\alpha$ gel) with a higher alcohol and/or a higher fatty acid that is a general formulation component and a cationic surfactant using water as a medium, an interlayer spacing of the lamellar structure narrows. Therefore, the gel has very high viscosity by two-fold concentration or so, and it is difficult to stir and mix the components upon production of a composition. With the two-fold concentration or so and the small amount of the production amount, it is not impossible to stir and mix against viscosity resistance caused by such low water. However, if a further highly concentrated hair conditioning composition is produced on a large scale, it is bound to require at least a huge amount of energy for stirring and mixing, and it is very difficult to stably produce a composition in which components are homogeneously mixed.
On the other hand, the hair conditioning composition of the present invention is a composition containing a polyhydric alcohol and/or polyethylene glycol having a melting point of 155°C or less as a medium in place of the water, wherein the endothermic peak of a gel which is formed from the higher alcohol and/or higher fatty acid and a cationic surfactant in the composition is 50°C or more as measured by a differential scanning calorimeter (DSC). According to the present invention, heated and melted polyhydric alcohol and/or polyethylene glycol is stirred and mixed with previously heated and melted other components under heating, thereafter the mixture is cooled, whereby homogeneous mixture can be obtained without generating a marked viscosity resistance as in the case of using water as a medium.
In addition, the hair conditioning composition, while having very low hygroscopicity during storage, uses specific polyhydric alcohol and/or a polyethylene glycol having a molecular weight within a specific range, thereby immediately absorbing water once water is added and turning into a conventional gel-type hair conditioner. In other words, the hair conditioning composition of the present invention encompasses an embodiment with an excellent texture both during storage and during use.
Hereinbelow, the present invention will be described in detail.
First, the essential components of the present invention: (a) one or more components selected from higher alcohols, higher fatty acids, and derivatives thereof, (b) a cationic surfactant, and (c) a polyhydric alcohol and/or polyethylene glycol, will be described.

(a) One or More Components Selected from Higher Alcohols, Higher Fatty Acids, and Derivatives Thereof

The hair conditioning composition of the present invention contains one or more components selected from higher alcohols, higher fatty acids, and derivatives thereof according to claim 1.
As the higher alcohols contained in the present invention, those normally used in cosmetics, pharmaceuticals, and the like can be used. Examples of the higher alcohols include straight chain alcohols (for example, lauryl alcohol, cetyl alcohol, stearyl alcohol, behenyl alcohol, myristyl alcohol, oleyl alcohol, cetostearyl alcohol, hardened rapeseed

oil alcohol, and the like); and branched-chain alcohols (for example, monostearyl glyceryl ether (batyl alcohol), 2-decyltetradecynol, lanolin alcohol, cholesterol, phytosterol, hexyldodecanol, isostearyl alcohol, octyldodecanol, and the like).

In the present invention, the straight chain alcohols having 16 or more carbon atoms are used, and the straight chain alcohols having 16 to 22 carbon atoms such as stearyl alcohol, behenyl alcohol, oleyl alcohol, cetostearyl alcohol, and cetyl alcohol can be preferably used.

In addition, the derivatives of higher alcohols contained in the present invention are a compound represented by the following formula (I).

$$R^1\text{-O-(-(}CH_2)_y\text{-O-)}_x\text{-H} \qquad (I)$$

In the formula (I), $R^1$ is a straight chain or branched fatty alcohol residue having 10 to 24 carbon atoms, and each of x and y is an integer of 1 to 3. Examples of such compounds include polyoxyethylene (1) stearyl alcohol, polyoxyethylene (2) cetostearyl alcohol, polyoxypropylene (3) lauryl alcohol, and polyoxybutylene (2) cetyl alcohol.

In addition, as the higher fatty acids contained in the present invention, those normally used in cosmetics, pharmaceuticals, and the like can be also used. As the higher fatty acids, the higher fatty acids having 12 to 22 carbon atoms are used, and examples include lauric acid, myristic acid, palmitic acid, stearic acid, behenic acid, oleic acid, undecylenic acid, tall acid, isostearic acid, linolic acid, linoleic acid, eicosapentaenoic acid (EPA), docosahexaenoic acid (DHA), and palm oil fatty acid. These can be used alone or as a combination of two or more kinds. Particularly, in the present invention, the straight chain fatty acids having 16 or more carbon atoms are preferable, and the straight chain fatty acids having 16 to 22 carbon atoms such as palmitic acid, stearic acid, and behenic acid can be particularly preferably used.

In addition, the derivatives of higher fatty acids contained in the present invention are a compound represented by the following formula (II).

$$R^2\text{-COO-}CH_2\text{-CH(OH)-}CH_2\text{-Q} \qquad (II)$$

In the formula (II), $R^2$ is a straight chain or branched fatty acid residue having 9 to 23 carbon atoms, and Q is H or OH. Examples of such compounds include monoglyceride stearate, propylene glycol monostearate, and monoglyceride oleate.

The hair conditioning composition of the present invention can contain the higher alcohols, the higher fatty acids, and/or derivatives thereof alone or as a combination of two or more kinds. Particularly, in the present invention, it is preferred that a higher alcohol and/or a higher alcohol derivative having a straight chain fatty acid residue having 16 to 22 carbon atoms is contained as the component (a).

(a) One or more components selected from higher alcohols, higher fatty acids, and derivatives thereof, in the hair conditioning composition of the present invention, can be contained in the range of 10 to 90% by mass and more preferably 20 to 50% by mass based on the composition, while it also depends on the amount of (b) cationic surfactant contained. When the amount of the component (a) contained is less than 10% by mass, energy reduction during production and transfer is not enough, and also viscoelasticity in the composition after dilution may be impaired.

(b) Cationic Surfactant

The hair conditioning composition of the present invention contains a cationic surfactant.

As the cationic surfactant contained in the present invention, those normally used in cosmetics, pharmaceuticals, and the like can be used. Particularly, a mono long-chain alkyl type quaternary ammonium salt represented by the following formula (III) is preferably used.

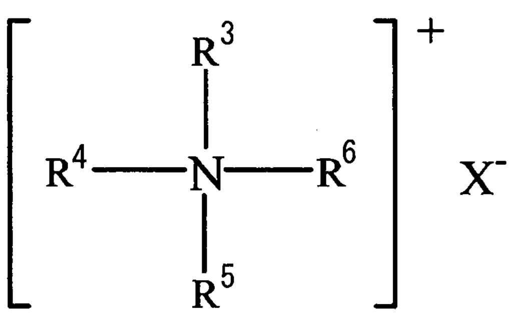

(III)

In the formula (III), $R^3$ represents a straight chain or branched alkyl group having 8 to 36 carbon atoms that may be substituted with a hydroxyl group, or $R^7$-$(Z1)_q$-$(Y1)_p$-.

$R^7$ represents a straight chain or branched alkyl group having 8 to 36 carbon atoms that may be substituted with a hydroxyl group, Y1 represents a linkage group selected from $-CH_2CH_2-$, $-CH_2CH_2CH_2-$, $-CH_2CH_2O-$, and $-CH(OH)CH_2O-$, and Z1 represents a linkage group selected from an amide bond (-CONH-), ether bond (-O-), and ester bond (-COO-). Each of p and q represents an integer of 0 or 1.

In addition, $R^4$, $R^5$ and $R^6$ in the formula (III) represent an alkyl group having 1 to 3 carbon atoms or benzyl group that may be substituted with a hydroxyl group and may be the same or different. X represents a halogen atom, an alkyl sulfate group having 1 or 2 carbon atoms, or anion that may form a salt with quaternary ammonium such as a residue in which a hydrogen atom of an organic acid is removed.

Examples of the mono long-chain alkyl type quaternary ammonium salts represented by the formula (III) are lauryl trimethyl ammonium chloride, lauryl trimethyl ammonium bromide, myristyl trimethyl ammonium chloride, myristyl trimethyl ammonium bromide, cetyl trimethyl ammonium chloride, cetyl trimethyl ammonium bromide, stearyl trimethyl ammonium chloride, stearyl trimethyl ammonium bromide, behenyl trimethyl ammonium chloride, behenyl trimethyl ammonium bromide, cetyltrimethylammonium methanesulfonate, stearyltrimethylammonium methosulfate, myristyldimethylbenzylammonium chloride, cetyldimethylbenzylammonium chloride, stearyldimethylbenzylammonium chloride, octyldihydroxyethylmethylammonium chloride, 2-decyltetradecyltrimethylammonium chloride, 2-dodecylhexadecyltrimethylammonium chloride, stearoxypropyltrimethylammonium chloride, and stearyl PG trimethylamine bromide.

Also, in the present invention, as a component to form the mono long-chain alkyl type quaternary ammonium salts of the formula (III), the mono long-chain alkyl type amine represented by the following formula (IV) and an organic acid can be contained in combination.

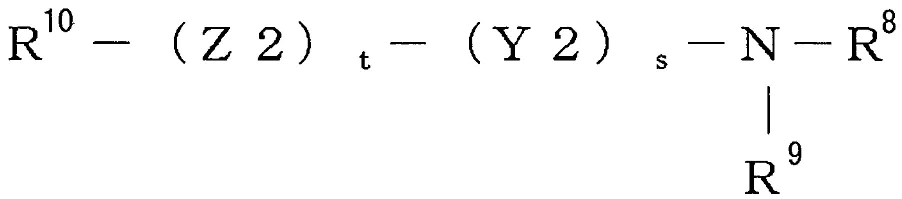

(IV)

In the formula (IV), $R^8$ and $R^9$ represent an alkyl group having 1 to 3 carbon atoms or benzyl group that may be substituted with a hydroxyl group and may be the same or different. $R^{10}$ represents a straight chain or branched alkyl group having 8 to 36 carbon atoms that may be substituted with a hydroxyl group.

In addition, Y2 represents a linkage group selected from $-CH_2CH_2-$, $-CH_2CH_2CH_2-$, $-CH_2CH_2O-$, and $-CH(OH)CH_2O-$, and Z2 represents a linkage group selected from an amide bond (-CONH-), ether bond (-O-), and ester bond (-COO-). Each of s and t represents an integer of 0 or 1.

As the organic acid combined with the mono long-chain alkyl type amine, for example, water soluble organic acids such as succinic acid, DL-malic acid, citric acid, tartaric acid, L-glutamic acid, lactic acid, hydroxyethane diphosphonic acid, and edetic acid can be preferably used.

Examples of the combinations of the mono long-chain alkyl type amine represented by the formula (IV) and the organic acid suitable for the formulation into the present invention include dimethylamide propylamide stearate-glutamic acid, diethylamide propylamide stearate-lactic acid, stearoxypropyl dimethylamide-malic acid, stearyl PG dimethylamine-glutamic acid, behenamidopropyl dimethylamine-succinic acid, and stearamidopropyl dimethanolamine-tartaric acid.

The hair conditioning composition of the present invention can contain the cationic surfactant alone or as a combination of two or more kinds.

The amount of (b) cationic surfactant contained in the composition of the present invention is from 5 to 35% by mass and more preferably from 10 to 25% by mass based on the composition. When the amount of the component (b) contained is less than 5% by mass, texture and viscoelasticity in the composition after dilution may be insufficient, and energy reduction during production and transfer is not also sufficient. When the amount of the component (b) contained exceeds 40% by mass, stickiness may be caused in the composition, and the composition may not be homogeneously diluted. Also, skin irritation may be caused by the high formulation of the surfactant.

(c) Polyhydric Alcohol and/or Polyethylene Glycol

The hair conditioning composition of the present invention contains a water-soluble polyhydric alcohol and/or polyethylene glycol according to claim 1 as a medium for the components.

In the temperature-rise measurement (heating rate of 2.0°C/min) using a differential scanning calorimeter

(DSC6100, manufactured by SII Nanotechnologies, Inc.), the polyhydric alcohol and/or polyethylene glycol contained in the present invention has an endothermic peak showing a melting point of the single body of 155°C or less. Also, the polyhydric alcohol and/or polyethylene glycol contained in the present invention is a medium that makes the endothermic peak of a gel which is formed from (a) and (b) in the composition 50°C or more as measured by a differential scanning calorimeter (DSC), when the composition of the present invention is subjected to the temperature-rise measurement.

By using a medium with such characteristics, it is possible to homogeneously stir and mix the formulation components under heating and melting, and a dilutable concentrated hair conditioning composition that is a solid or semisolid at ambient temperature less than 50°C can be obtained.

When a polyhydric alcohol and/or polyethylene glycol alone has a melting point of not less than 155°C, it is not preferred since other melting components may undergo heat degradation upon mixing with the polyhydric alcohol and/or polyethylene glycol melted during production.

Considering storage stability and ease of handling after dilution as a hair conditioning composition, the polyhydric alcohol and/or polyethylene glycol having high viscosity (preferably 50,000 Pa·s or more) or becoming a solid in the temperature range that the storage or use of the composition is supposed (less than 50°C) is more preferable. Furthermore, in view of water absorbability of the composition after dilution, the polyhydric alcohol and/or polyethylene glycol is preferably soluble in water at less than 50°C for 20% by mass or more.

Examples of the polyhydric alcohol used in the present invention include erythritol, maltitol, sorbitol and xylitol.

The polyethylene glycol includes a polyethylene glycol having a molecular weight of 300 to 5,000,000, and the like.

The hair conditioning composition of the present invention can contain the polyhydric alcohol and polyethylene glycol alone or as a combination of two or more kinds. Particularly, in being capable of producing the hair conditioning composition as powder or solid, and in being capable of providing low hygroscopicity and high water absorbability to the composition, erythritol, maltitol, and/or a polyethylene glycol having a molecular weight of 3,000 to 300,000 are preferably used, and erythritol is particularly preferably used.

Sugars such as sucrose, fructose, lactose, xylose, mannose, and ribulose are not preferred as the medium for the present invention since the sugars cause browning at high temperature and condense.

In the hair conditioning composition of the present invention, (c) polyhydric alcohol and/or polyethylene glycol according to claim 1 can be contained in the range of 10 to 90% by mass based on the composition. When the amount of the component (c) contained is less than 10% by mass, homogeneous mixing of the formulation components and water absorbability of the composition may be insufficient.

Next, the compounding ratio of the essential components will be described.

In the hair conditioning composition of the present invention, the molar ratio of (a) one or more components selected from higher alcohols, higher fatty acids, and derivatives thereof to (b) cationic surfactant ((a)/(b)) is 2.5 or more to less than 6.0 and preferably 2.5 or more to 5.0 or less.

When the molar ratio is less than 2.5, in other words, the ratio of the cationic surfactant is high, hygroscopicity of the hair conditioning composition increases, and stickiness may be caused during use. Furthermore, water absorbability of the composition is reduced, and the separation of the composition (gel) from water may be caused when diluted. Also, with the increase in the amount of the cationic surfactant contained, skin irritation may increase.

When the molar ratio is 6.0 or more, in other words, the ratio of the one or more components selected from higher alcohols, higher fatty acids, and derivatives thereof is too high, the one or more components selected from higher alcohols, higher fatty acids, and derivatives thereof are likely to deposit in the hair conditioning composition or diluted product thereof.

The hair conditioning composition of the present invention can be easily produced by melting and mixing (a) one or more components selected from higher alcohols, higher fatty acids, and derivatives thereof and (b) cationic surfactant under heating, mixing the mixture with separately melted (c) polyhydric alcohol and/or polyethylene glycol under heating, and thereafter cooling the mixture to room temperature. The temperature where the polyhydric alcohol and/or polyethylene glycol is heated and mixed with other components is not less than the melting temperature of a gel with the components and is from 50 to 155°C that is the temperature not less than the melting point of the polyhydric alcohol and/or polyethylene glycol. In other words, in the temperature range, the components of (a) to (c) are all in the melted state that can be mixed and also do not cause heat degradation. Furthermore, the components (a) to (c) in the temperature range are mixed, and thereafter, this mixture was cooled to less than 50°C, whereby a solid or semisolid composition which is excellent in ease of handling can be obtained.

Hereinbelow, the production example of the hair conditioning composition of the present invention is described.

(Production Example of Hair Conditioning Composition)

A cationic surfactant and one or more components selected from higher alcohols, higher fatty acids, and derivatives thereof are melted and mixed under heating at 130°C. In addition, a polyhydric alcohol and/or polyethylene

glycol is heated to 130°C or the melting point of the polyhydric alcohol and/or polyethylene glycol, whichever is higher. The melted mixture of the cationic surfactant and the one or more components selected from higher alcohols, higher fatty acids, and derivatives thereof is added thereto and mixed until being homogeneous while stirring, and thereafter, the resulting mixture is entirely cooled to 20 to 40°C, to obtain a hair conditioning composition. In the case where the composition is solid, the composition may be pulverized with a pulverizer as necessary.

The production example is not to limit the production conditions of the hair conditioning composition of the present invention, and for example, an optional component other than the essential components (a) to (c) can be also added to produce the composition. However, it is preferred to conduct the melting and mixing of the essential components (a) and (b). In the case of the composition obtained by pulverizing and mixing the essential components (a) to (c) without melting and mixing under heating, and molding the mixture, the effects tend to be insufficient as compared to those obtained by melting and mixing the components.

The hair conditioning composition of the present invention can be any dosage form depending on the desired product form, such as solid compositions with any size and shape, powdered compositions such as powder, granules or flakes, or concentrated liquid compositions. Examples of the product forms of the hair conditioning composition of the present invention include various forms depending on use conditions and the like, such as a form in which the powdered hair conditioning composition is individually-packaged by the amount used, a form in which the powdered composition is filled in a bottle and the required amount is taken out when used, a form in which the solid (bar) composition is put in a container when used and diluted, and are not particularly limited as far as it does not impair the effects of the present invention. In addition, in the industrial production of the conventional hair conditioner, the process of mixing the hair conditioner composition of the present invention with water can be applied.

As described above, it is particularly preferred that the hair conditioning composition of the present invention is used as a hair conditioning precursor composition before being applied to hair as a hair conditioner. In other words, the hair conditioning composition of the present invention can be used in the same manner as the conventional hair conditioner by diluting the appropriate amount with water or the like when used. While the specific dilution rate in the present invention can be properly adjusted by the amounts of the essential components contained and the compounding ratio thereof, it is normally preferred that the composition of the present invention is diluted with water in an amount of 3 to 15 times by mass. The higher the temperature of dilution water is, the higher the dilution rate is, and even water at room temperature (20 to 30°C) can sufficiently dilute the composition. In addition, the lower (less than 200 ppm) the hardness of dilution water is, the higher the dilution rate is, and even water with high hardness can sufficiently dilute the composition depending on a dilution method.

Also, the hair conditioning composition of the present invention may be used after diluting the required amount for each use on hands or heads or may be previously diluted in a container with an appropriate size in a mass and used as the conventional conditioner.

The hair conditioner composition of the present invention can contain other components normally used in cosmetics, pharmaceuticals, and the like within a range which does not impair the effects of the present invention, in addition to the essential components.

For example, while the hair conditioning composition of the present invention can contain water, the amount contained is 10% by mass or less, preferably 7% by mass or less, and further preferably 5% by mass or less, based on the composition of the present invention. Furthermore, in the present invention, it is most preferable that water is not substantially contained. When the amount of water contained exceeds 10% by mass, during the production of the composition, lowering of stirring and mixing properties due to viscosity increase and scattering of the components due to boiling may be caused. In addition, the formulation of excess water is not preferred since not only the solution of the problem in the present invention is insufficient, but also stickiness may be caused upon packaging and opening of the composition, and the transfer to a container and the like may be prevented.

Examples of other components which can be contained within the range that the effects of the present invention is not impaired include oils, amphoteric surfactants, nonionic surfactants, moisturizers, thickeners, coating agents, UV absorbers, metal ion sequestering agents, pH adjusters, skin nutrients, vitamins, antioxidants, antioxidant aids, and perfumes.

Examples of oils include liquid oils, solid oils, hydrocarbon oils, and silicone oils.

Examples of liquid oils include avocado oil, camellia oil, turtle oil, macadamia nut oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, paulownia oil, Japanese tung oil, jojoba oil, germ oil, and triglycerin.

Examples of solid oils include cacao butter, coconut oil, horse fat, hardened coconut oil, palm oil, beef tallow, mutton tallow, hardened beef tallow, palm kernel oil, pork tallow, beef bone tallow, Japan wax kernel oil, hardened oil, heatsfoot oil, Japan wax, and hardened castor oil.

Examples of hydrocarbon oils include liquid paraffin, ozokerite, squalane, pristane, paraffin, ceresin, squalene, petrolatum, and microcrystalline wax.

Examples of silicone oils include linear polysiloxanes (such as dimethylpolysiloxane, methylphenylpolysiloxane, and diphenylpolysiloxane); cyclic polysiloxanes (such as octamethylcyclotetrasiloxane, decamethylcyclopentasiloxane, and dodecamethylcyclohexasiloxane); silicon resin forming three-dimensional network structure; silicone rubber; various kinds of modified polysiloxane (such as amino modified polysiloxane, polyether modified polysiloxane, alkyl modified polysiloxane, polyether/alkyl co-modified polysiloxane, fluorine modified polysiloxane, polyoxyethylene/polyoxypropylene copolymer modified polysiloxane, linear amino polyether modified polysiloxane, amidoalkyl modified polysiloxane, aminoglycol modified polysiloxane, aminophenyl modified polysiloxane, carbinol modified polysiloxane, polyglycerin modified polysiloxane, and polyglycerin/alkyl co-modified polysiloxane); dimethiconol; and acrylic silicones.

Examples of lipophilic nonionic surfactants include sorbitan fatty acid esters (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerol or polyglycerol fatty acid esters (such as glycerol mono-cotton seed oil fatty acid ester, glycerol monoerucate, glycerol sesquioleate, glycerol monostearate, glycerol $\alpha$, $\alpha$'-oleate pyroglutamate, and glycerol monostearate malate); propylene glycol fatty acid esters (such as propylene glycol monostearate); hardened castor oil derivatives; and glycerol alkyl ethers.

Examples of hydrophilic nonionic surfactants include POE-sorbitan fatty acid esters (such as POE-sorbitan monooleate, POE-sorbitan monostearate, and POE-sorbitan tetraoleate); POE sorbitol fatty acid esters (such as POE-sorbitol monolaurate, POE-sorbitol monooleate, POE-sorbitol pentaoleate, and POE-sorbitol monostearate); POE-glycerol fatty acid esters (such as POE-monooleates, POE-glycerol monostearate, POE-glycerol monoisostearate, and POE-glycerol triisostearate); POE-fatty acid esters (such as POE-distearate, POE-monodioleate, and ethylene glycol distearate); POE-alkyl ethers (such as POE-lauryl ether, POE-oleyl ether, POE-stearyl ether, POE-behenyl ether, POE-2-octyldodecyl ether, and POE-cholestanol ether); Pluronic type surfactants (such as Pluronic); POE/POP-alkyl ethers (such as POE/POP cetyl ether, POE/POP 2-decyltetradecyl ether, POE/POP monobutyl ether, POE/POP hydrogenated lanolin, and POE/POP glycerol ether); tetra POE/tetra POP-ethylenediamine condensates (such as Tetronic); POE-castor oil or hardened castor oil derivatives (such as POE-castor oil, POE-hardened castor oil, POE-hardened castor oil monoisostearate, POE- hardened castor oil triisostearate, POE-hardened castor oil monopyroglutamate monoisostearate diester, and POE-hardened castor oil maleate); POE-beeswax lanolin derivatives (such as POE-sorbitol beeswax); alkanolamides (such as coconut oil fatty acid diethanolamide, lauric acid monoethanolamide, and fatty acid isopropanolamide); POE-propylene glycol fatty acid esters; POE-alkylamines; POE-fatty acid amides; sucrose fatty acid esters; alkylethoxydimethylamine oxides; and trioleyl phosphate.

Examples of semi-synthetic water-soluble polymers include starch polymers (such as carboxymethyl starch and methylhydroxypropyl starch); cellulose polymers (such as methyl cellulose, ethyl cellulose, methylhydroxypropyl cellulose, hydroxyethyl cellulose, sodium cellulose sulfate, dialkyldimethylammonium sulfate cellulose, hydroxypropyl cellulose, carboxymethyl cellulose, sodium carboxymethyl cellulose, crystalline cellulose, cellulose powder, hydrophobically modified compounds of these polymers (e.g., partially stearoxy modified compounds), and cation modified compounds of these polymers); alginate polymers (such as sodium alginate and propylene glycol alginate); and sodium pectate.

Examples of synthetic water-soluble polymers include vinyl polymers (such as polyvinyl alcohol, polyvinyl methyl ether, polyvinylpyrrolidone, and carboxyvinyl polymer); polyoxyethylene polymers (such as polyoxyethylene/polyoxypropylene copolymers, for example, polyethylene glycol 20,000, 40,000 or 60,000); poly(dimethyldiallylammonium halide) type cationic polymers (such as Merquat100 manufactured by Merck & Co., Inc.); dimethyldiallylammonium halide/acrylamido copolymer type cationic polymers (such as Merquat550 manufactured by Merck & Co., Inc.); acrylic polymers (such as sodium polyacrylate, polyethyl acrylate, and polyacrylamide); polyethyleneimine; cationic polymers; magnesium aluminum silicate (veegum); polyquaternium-39; polyquaternium-47; polyquaternium-74; and (propyltrimonium chloride acrylamide/dimethyl acrylamide) copolymer.

Examples of UV absorbers include benzoic acid UV absorbers (such as p-aminobenzoic acid (hereinafter abbreviated as PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester); anthranilic acid UV absorbers (such as homomenthyl N-acetylanthranilate); salicylic acid UV absorbers (such as amyl salicylate, menthyl salicylate, homomenthyl salicylate, octyl salicylate, phenyl salicylate, benzyl salicylate, and p-isopropanolphenyl salicylate); cinnamic acid UV absorbers (such as octyl cinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl $\alpha$-cyano-$\beta$-phenylcinnamate, 2-ethylhexyl $\alpha$-cyano-$\beta$-phenylcinnamate, and glyceryl mono-2-ethylhexanoyl-diparamethoxy cinnamate); benzophenone UV absorbers (such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone); 3-(4'-methylbenzylidene)-d,l-camphor and 3-benzylidene-d,l-camphor; 2-phenyl-5-methylbenzoxazol; 2,2'-hydroxy-5-methylphenylbenzotriazol; 2-(2'-hydroxy-5'-t-octylphenyl) benzotriazol; 2-(2'-hydroxy-5'-methylphenylbenzotriazol; dianisoylmethane; 4-methoxy-4'-t-butyldibenzoylmethane; 5-(3,3-dimethyl-2-norbornylidene)-3-pentane-2-one; and triazine UV absorbers (such as 2-4[(2-hydroxy-3-dodecyloxypropyl)oxy]-2-hydroxyphenyl)-4,6-bis(2,4-dimethylphenyl)-1,3, 5-triazine and 2-4[(2-hydroxy-3-tridecyloxypropyl)oxy]-2-hydroxyphenyl)-4,6-bis(2,4-dimethylphenyl)-1,3, 5-triazine).

Examples of metal ion sequestering agents include 1-hydroxyethane-1,1-diphosphonic acid, 1-hydroxyethane-1,1-diphosphonic acid 4Na salt, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of pH adjusters include buffers such as lactic acid/sodium lactate, citric acid/sodium citrate, and succinic acid/sodium succinate.

Examples of vitamins include vitamins A, B1, B2, B6, C, and E and the derivatives thereof; pantothenic acid and the derivatives thereof; and biotin.

Examples of antioxidants include tocopherols, dibutylhydroxytoluene, butylhydroxyanisole, and gallic acid esters.

Examples of other components which can be contained include antiseptic (such as ethylparaben, butylparaben, 1,2-alkane diol (having a carbon chain length of 6 to 14) and the derivatives thereof, phenoxyethanol, and methylchloroisothiazolinone); antiphlogistic (such as glycyrrhizic acid derivatives, glycyrrhetinic acid derivatives, salicylic acid derivatives, hinokitiol, zinc oxide, and allantoin); whitening agent (such as saxifrage sarmentosa extract and arbutin); various extracts (such as phellodendron bark, goldthread, lithospermum root, paeonia albiflora, swertia japonica, birch, sage, loquat, carrot, aloe, malva sylvestris (mallow), iris, vitis vinifera (grape), coix lacryma-jobi (job's tears), luffa cylindrica, lily, saffron, cnidium officinale, ginger, hypericum perforatum, ononis spinosa, allium sativum (gerlic), capsicum frutescens, citrus unshiu peel, angelica acutiloba, and sea alga); activator agent (such as royal jelly, photosensitizers, and cholesterol derivatives); blood circulation accelerator (such as nonylic acid vanillylamide, nicotinic acid benzyl esters, nicotinic acid $\beta$-butoxy ethyl esters, capsaicin, Zingerone, Cantharides tincture, ichthammol, tannic acid, $\alpha$-borneol, tocopherol nicotinate, inositol hexanicotinate, cyclandelate, cinnarizine, tolazoline, acetylcholine, verapamil, cepharanthine, and $\gamma$-orizanol); antiseborrheic agent (such as sulfur and thianthol); anti-inflammatory agent (such as tranexamic acid, thiotaurine, and hypotaurine); and aromatic alcohols (such as benzyl alcohol and benzyloxy ethanol).

The hair conditioner in the present invention refers to overall cosmetics that provide conditioning effect to hair, and examples include hair rinse, hair treatment, and hair pack. The hair conditioner can include both type of being applied to hair and spread well overall when used and thereafter washed out (rinsed) with hot water, water or the like, and type of not being washed out after application.

Hereinbelow, the present invention is described in more detail with reference to Examples. However, the present invention is not limited to these Examples. The amount contained is all shown in % by mass unless otherwise described.

EXAMPLES

<Selection of Medium>

The mediums preferred in a hair conditioning composition were studied. The method for preparing the composition of each test example and the evaluation methods (DSC, hygroscopicity, and water absorbability) are as follows.

<u>Preparation of Hair Conditioning Composition</u>

A mixture obtained by melting and stirring 13.9% by mass of stearyl trimethyl ammonium chloride and 32.6% by mass of stearyl alcohol (molar ratio of both: 3) under heating at 130°C and 53.5% by mass of each medium shown in the following Table 1 previously melted were mixed under heating until homogeneous. Thereafter, the mixture was transferred to a container and cooled to room temperature to give a hair conditioning composition. These hair conditioning compositions are compositions in which general hair conditioners are concentrated into one-tenth and can be diluted with water or the like as a hair conditioning precursor composition and used.

<u>Method for Evaluating Hair Conditioning Composition</u>

(Determination of DSC Endothermic Peak Temperature)

10 mg of each hair conditioning composition and 10 mg of dimethicone (20cs) as a reference substance were each enclosed in an Ag closed cell and set to a holder of a differential scanning calorimeter (DSC6100, manufactured by SII Nanotechnologies, Inc.). The sample was heated from 30 to 200°C at a heating rate of 2.0°C/min, and a temperature

where heat absorption generated when the phase state of the composition changes was the maximum was recorded as a DSC endothermic peak temperature.

When a plurality of peak temperatures were obtained from the composition, based on the endothermic peak temperature of the contained component alone and the results of X-ray diffraction of the composition at each temperature, the peak temperatures were divided into DSC endothermic peak temperature derived from a medium, DSC endothermic peak temperature derived from a gel which was formed from a cationic surfactant and a higher alcohol, and DSC endothermic peak temperature of a higher alcohol which caused phase separation without contributing to the gel formation.

Among them, the endothermic peak temperatures derived from each medium and gel and the evaluations of the compositions by the following evaluation criteria are shown in Table 1.

(Evaluation Criteria of Composition)

O: The DSC endothermic peak temperature derived from a gel which is formed from the cationic surfactant and the higher alcohol in the composition is 50°C or more, and the endothermic peak temperature showing the melting point of the medium is 155°C or less. X: The DSC endothermic peak temperature derived from a gel which is formed from the cationic surfactant and the higher alcohol in the composition is less than 50°C, or the endothermic peak temperature showing the melting point of the medium is over 155°C.

Method for Evaluating Hygroscopicity

Each hair conditioning composition was spread all over a weighing dish and was each allowed to stand still under conditions at a temperature of 45°C and a relative humidity of 75, 80, 85, or 90% for 6 hours. The result of evaluating the rate of weight change of each composition in each humidity condition as hygroscopicity is shown in Table 1.

$$\text{Rate of Weight Change} = (\text{Weight after Test} - \text{Weight Before Test})/(\text{Weight Before Test}) \times 100\ (\%)$$

Method for Evaluating Water Absorbability

The appropriate amount of each hair conditioning composition was put in a mesh bag, and the bag was immersed into water. The dilution rates (Weight after Test/Weight Before Test) at 3 hours and 24 hours after the immersion were determined. The result of evaluating the dilution rates of each composition during each immersion time as water absorbability of the compositions are shown in Table 1. The target values in Table 1 show the standard dilution rates in the actual use of the hair conditioning compositions prepared as above and are based on the amount of the cationic surfactant contained.

Table 1

| Kinds of Medium | | Erythritol | Maltitol | Sorbitol | D-mannite | Xylitol | Polyethylene glycol 6000 | Polyethylene glycol 400 | Glycerin | Propylene glycol | Isopentyldiol | 1,3-Butylene glycol | Dipropylene glycol |
|---|---|---|---|---|---|---|---|---|---|---|---|---|---|
| DSC Endothermic Peak Temperature (°C) | Gel | 72.3 | 74.6 | 73.2 | 75.2 | 73.2 | 72.4 | 66.6 | 63.8 | 45.4 | 43.0 | 47.4 | 43.5 |
| | Medium | 116.1 | 136.5 | 67.9 | 162.3 | 88.1 | 59.6 | - | - | - | - | - | - |
| | Evaluation | ○ | ○ | ○ | × | ○ | ○ | ○ | ○ | × | × | × | × |
| Hygroscopicity (Rate of Increased Weight after 6 Hours) | 90%RH | 5.9% | 7.0% | 28.2% | 3.5% | 31.0% | 6.0% | 17.8% | 11.3% | 21.3% | 10.3% | 17.2% | 16.2% |
| | 85%RH | 3.5% | 3.0% | 17.7% | 3.0% | 20.4% | 2.5% | 10.3% | 5.4% | 10.0% | 6.5% | 9.4% | 9.5% |
| | 80%RH | 1.5% | 3.0% | 12.4% | 1.5% | 2.5% | 1.5% | 6.5% | 4.0% | 2.0% | 4.5% | 4.4% | 6.9% |
| | 75%RH | 1.5% | 1.5% | 3.9% | 2.0% | 2.0% | 0.5% | 2.0% | 2.5% | 3.4% | 2.5% | 1.5% | 4.0% |
| Water Absorbability (Dilution Rate after a Given Time) | 3Hours Later | 8.4 | 8.1 | 10.0 | 7.2 | 8.1 | 8.6 | 9.0 | 4.5 | 6.5 | 4.8 | 5.0 | 7.5 |
| | 24 Hours Later | 13.6 | 13.6 | 14.0 | 12.5 | 13.7 | 12.2 | 12.0 | 7.5 | 13.0 | 11.7 | 12.0 | 14.3 |
| | Target Value | 10 | 10 | 10 | 10 | 10 | 10 | 10 | 10 | 10 | 10 | 10 | 10 |

According to the evaluation of the DSC endothermic peak temperatures in Table 1, the compositions using as a medium a dihydric alcohol with a low melting point such as propylene glycol, isopentyldiol, 1,3-butylene glycol, or dipropylene glycol changed from a gel state to a liquid state with high viscosity at 40 to 50°C and had uncomfortable stickiness at ambient temperature. Therefore, it is obvious that these compositions using a dihydric alcohol as a medium and having a gel melting point around the temperature for using or storing a hair conditioner do not satisfy the stability of the product as a hair conditioning composition and ease of handling.

On the other hand, the compositions using as a medium erythritol, maltitol, sorbitol, D-mannit, xylitol, glycerin, or polyethylene glycol all showed endothermic peak temperatures showing a gel melting at around 70°C. Among them, erythritol, maltitol, sorbitol, xylitol, and polyethylene glycol with high molecular weight (6,000) had the endothermic peak showing the melting point of a medium was higher than the melting temperature of a gel and had a solid state that was easy to handle at less than 50°C. In addition, the compositions using glycerin and polyethylene glycol with low molecular weight ((average molecular weight of 400) were agents having a semi-solid state at ambient temperature, that were excellent in ease of handling.

However, when D-mannit with a very high melting point was used as a medium, a part of the components pyrolyzed at melting and mixing, and a normal composition could not obtained.

In view of the above, in the determination of the DSC endothermic peak temperature, it is obvious that the endothermic peak temperature derived from a gel which is formed from the cationic surfactant and the higher alcohol is made at 50°C or more, and polyhydric alcohol or polyethylene glycol alone having a melting point of 155°C or less is used as a medium, whereby a hair conditioning composition having excellent stirring and mixing properties in the production at low water content, that is excellent in ease of handling is obtained.

In addition, according to the evaluation of hygroscopicity in Table 1, in the compositions using a dihydric alcohol or low molecular weight polyethylene glycol, the higher the storage humidity becomes, the more the rate of weight increases, and at a relative humidity of 90%, a weight increase close to 20% was found. Also in xylitol and sorbitol, particularly marked hygroscopicity was found in high humidity conditions, and also in glycerin, the rate of weight increase slightly increased at a relative humidity of 90%.

On the other hand, erythritol, maltitol, D-mannit, or high molecular weight polyethylene glycol was used as a medium, the rates of weight increase did not reach to 10% in all humidity conditions, and very low hygroscopicity was shown.

Furthermore, according to the evaluation of water absorbability in Table 1, the compositions using compounds other than glycerin as a medium all showed high water absorbability and reached to 10 times as the dilution rate that was the target value within 24 hours.

Since the conditioning composition is supposed to be used or stored in high humidity conditions such as bathroom, it is preferred that hygroscopicity is low regardless of humidity change, and considering that the composition is diluted with water before use, it is also preferred that the composition rapidly absorbs water to be a state that can be used as a hair conditioner.

Therefore, in the hair conditioning composition of the present invention, from the viewpoint of hygroscopicity and water absorbability, erythritol, maltitol, and high molecular weight polyethylene glycol are particularly preferable as a medium. In addition, based on further studies, it was found that the molecular weight of polyethylene glycol comprising a composition excellent in ease of handling was from 3,000 to 300,000. In addition, it is obvious that such hair conditioning composition of the present invention has excellent characteristics as a hair conditioning composition to which water was added before use.

<Molar Ratio of Higher Alcohol to Cationic Surfactant>

The hair conditioning compositions with the formulation compositions shown in Table 2 were evaluated according to the above evaluation methods and the following evaluation methods. The evaluation results are shown in Table 2. These compositions are concentrated hair conditioners and can be diluted with water or the like as a hair conditioning precursor composition and used.

<u>Method for Evaluating Hair Conditioning Composition</u>

(Determination of DSC Endothermic Peak Temperature of 10 Times Diluted Product)

For the hair conditioners obtained by diluting each hair conditioning composition with 9 times amount of water (10 times diluted product of original compositions), the endothermic peak temperature at 30 to 90°C using the same apparatuses and methods as above was determined. Whether an endothermic peak other than a rinse gel at 30 to 90°C was generated was confirmed according to the determination, and the evaluation was carried out according to the following criteria. For the sample causing water discharge upon being diluted by 10 times, only a gel phase part was

collected to carry out the determinations and evaluations.

(DSC Evaluation Criteria of 10 Times Diluted Product)

○: An endothermic peak was not found other than the endothermic peak of a rinse gel.
X: An endothermic peak was found other than the endothermic peak of a rinse gel.

Method for Evaluating Hair Conditioning Properties

For the hair conditioners obtained by diluting each hair conditioner composition with 9 times amount of water (10 times diluted product of original compositions), an actual use test by 10 expert panels was conducted. In other words, each hair conditioner was applied to hair and washed with water, and thereafter conditioning properties of wet hair was evaluated with the following criteria. For the sample causing water discharge upon being diluted by 10 times, the evaluation was defined as x.

(Evaluation Criteria of Conditioning Properties)

O: 8 or more panels evaluated that hair was smooth during use.
Δ: 4 to 7 panels evaluated that hair was smooth during use.
X: 3 or less panels evaluated that hair was smooth during use.

Table 2

| | | Example 1 | Example 2 | Example 3 | Example 4 | Example 5 | Example 6 | Example 7 | Example 8 | Example 9 | Example 10 |
|---|---|---|---|---|---|---|---|---|---|---|---|
| Stearyl trimethyl ammonium chloride | | 36.0 | 28.1 | 19.5 | 17.0 | 15.0 | 13.4 | 12.2 | 10.2 | 8.8 | 7.8 |
| Stearyl alcohol | | 14.0 | 21.9 | 30.5 | 33.0 | 35.0 | 36.6 | 37.8 | 39.8 | 41.2 | 42.2 |
| Erythritol | | 50.0 | 50.0 | 50.0 | 50.0 | 50.0 | 50.0 | 50.0 | 50.0 | 50.0 | 50.0 |
| Molar Ratio of Higher Alcohol to Cationic Surfactant | | 0.5 | 1.0 | 2.0 | 2.5 | 3.0 | 3.5 | 4.0 | 5.0 | 6.0 | 7.0 |
| DSC Endothermic Peak Temperature [°C] | Gel | 71.9 | 78.4 | 73.7 | 72.8 | 72.3 | 71.3 | 71.1 | 69.9 | 68.7 | 68.6 |
| | Medium | 114.5 | 118.9 | 116.7 | 118.2 | 116.1 | 118.7 | 118.8 | 119.2 | 118.4 | 119.1 |
| | Evaluation | ○ | ○ | ○ | ○ | ○ | ○ | ○ | ○ | ○ | ○ |
| DSC of 10 Times Diluted Product | | × | ○ | ○ | ○ | ○ | ○ | ○ | ○ | × | × |
| Hygroscopicity (Rate of Increased Weight after 6 Hours) | 90%RH | 10.9% | 8.0% | 6.0% | 5.5% | 6.0% | 5.5% | 5.4% | 5.5% | 4.5% | 4.0% |
| | 85%RH | 5.4% | 3.5% | 3.5% | 2.5% | 3.0% | 2.5% | 2.5% | 2.5% | 1.5% | 1.5% |
| | 80%RH | 3.0% | 2.5% | 3.0% | 2.5% | 2.5% | 2.0% | 1.5% | 1.0% | 1.0% | 0.5% |
| | 75%RH | 2.0% | 2.5% | 2.5% | 1.5% | 1.5% | 1.0% | 1.5% | 0.5% | 0.5% | 0.5% |
| Water Absorbability (Dilution Rate after a Given Time) | 3 Hours Later | 5.1 | 3.1 | 7.5 | 11.5 | 10.9 | 10.9 | 11.2 | 9.7 | 8.0 | 8.5 |
| | 24 Hours Later | 4.6 | 2.4 | 11.8 | 15.9 | 15.6 | 14.6 | 13.6 | 12.2 | 10.5 | 10.1 |
| | Target Value | 22 | 18 | 13 | 11 | 10 | 9 | 8 | 7 | 6 | 5 |
| Conditioning Property | | × | × | Δ | ○ | ○ | ○ | ○ | ○ | Δ | × |

As shown in Table 2, in the hair conditioner compositions, both gel and medium showed an almost constant good endothermic peak temperature, regardless of the molar ratio of the higher alcohol (stearyl alcohol) to the cationic surfactant (stearyl trimethyl ammonium chloride). However, 10 times diluted product of each test sample caused an endothermic peak other than a rinse gel at a molar ratio of 0.5 or less and 6.0 or more, and such compositions were not homogeneous.

In addition, regarding hygroscopicity, when the molar ratio of the higher alcohol to the cationic surfactant was below 2.0, hygroscopicity was on the rise, and in the test example in which the molar ratio was 1.0 or less, hygroscopicity under high humidity was remarkable. On the other hand, in the test example in which the molar ratio was 2.0 or more, all maintained very low hygroscopicity.

In addition, regarding water absorbability, when the molar ratio of the higher alcohol to the cationic surfactant was 2.0 or less, the composition could not be diluted to the target value even after a period of 24 hours. On the other hand, in the test example in which the molar ratio was over 2.0, the composition was rapidly diluted and was excellent in water absorbability.

In addition, the conditioning properties of the 10 times diluted product were excellent in the test example with a molar ratio of the higher alcohol to the cationic surfactant of 2.5 to 5.0 in the composition and were likely to be poor in smoothness during use in the test example with a molar ratio of 2.0 or less and 6.0 or more.

Based on the above results, in the hair conditioning compositions of the present invention, it is preferred that the molar ratio of the higher alcohol to the cationic surfactant is 2.5 or more to less than 6.0.

<Dilution Rate of Hair Conditioner Composition>

The hair conditioning composition obtained by the following preparation method was diluted with water and defined as hair conditioners at dilution rates of 1.5 to 30. The results of evaluating these hair conditioners according to the following evaluation method and the above evaluation method (conditioning properties) are shown in Table 3.

<u>Preparation of Hair Conditioning Composition</u>

The mixture obtained by melting and stirring 15% by mass of stearyl trimethyl ammonium chloride and 35% by mass of stearyl alcohol under heating at 130°C and 50% by mass of erythritol previously melted were mixed under heating until homogeneous. Thereafter, the mixture was transferred to a container and cooled to room temperature, to give a hair conditioning composition. These compositions are concentrated hair conditioners and can be diluted with water or the like as a hair conditioning precursor composition and used.

<u>Method for Evaluating Hair Conditioner</u>

(Ease of Removal from Container)

Hair conditioner at each dilution rate was filled in a tube container, and ease of removing the product from a spout was determined by 10 panels and evaluated by the following criteria.

O: 8 or more panels evaluated that the product was easy to remove from a container and hard to drip from hands.
Δ: 4 to 7 panels evaluated that the product was easy to remove from a container and hard to drip from hands.
X: 3 or less panels evaluated that the product was easy to remove from a container and hard to drip from hands.

Table 3

| Dilution Rate | 1.5 | 2.5 | 3 | 5 | 10 | 15 | 30 |
|---|---|---|---|---|---|---|---|
| Ease of Removal from Container | × | Δ | ○ | ○ | ○ | ○ | Δ |
| Conditioning Property | ○ | ○ | ○ | ○ | ○ | ○ | × |

As shown in Table 3, when the dilution rate of the hair conditioning composition was 2.5 or less, gel dilution was insufficient, and thus, it was hard to remove from a container. In addition, when the dilution rate is 30 or more, hair conditioning components were too dilute, and conditioning properties declined.

Based on the above results, it is preferred that the dilution rates of the hair conditioning compositions of the present invention are 3 to 15.

<Type of Surfactant>

The hair conditioning compositions with the formulation compositions shown in Table 4 below were evaluated according to the above evaluation methods. The results are shown in Table 4.

Preparation of Hair Conditioning Composition

The mixture obtained by melting and stirring a surfactant and stearyl alcohol under heating at 130°C and erythritol previously dissolved were mixed under heating until homogeneous. Thereafter, the mixture was transferred to a container and cooled to room temperature, to give a hair conditioning composition. These compositions are concentrated hair conditioners and can be diluted with water or the like as a hair conditioning precursor composition and used.

Table 4

| | | Example 11 | Example 12 | Example 13 | Example 14 |
|---|---|---|---|---|---|
| Stearyl trimethyl ammonium chloride | | 17.4 | | | |
| Behenyl trimethyl ammonium chloride | | | 19.8 | | |
| Stearoxypropyltrimethylammonium chloride | | | | 16.7 | |
| Stearyl PG dimethylamine | | | | | 15.3 |
| Lactic acid | | | | | 3.5 |
| Stearyl alcohol | | 32.6 | 30.2 | 33.3 | 31.2 |
| Erythritol | | 50.0 | 50.0 | 50.0 | 50.0 |
| DSC Endothermic Peak Temperature [°C] | Gel | 72.3 | 79.1 | 70.9 | 61.0 |
| | Medium | 116.1 | 116.4 | 115.9 | 117.7 |
| | Evaluation | ○ | ○ | ○ | ○ |
| Hygroscopicity (Rate of Increased Weight after 6 Hours) | 90%RH | 6.0% | 5.0% | 5.9% | 7.5% |
| | 85%RH | 3.0% | 2.5% | 2.5% | 3.5% |
| | 80%RH | 2.5% | 2.5% | 1.5% | 2.0% |
| | 75%RH | 1.5% | 1.5% | 0.5% | 1.0% |
| Water Absorbability (Dilution Rate after a Given Time) | 3 Hours Later | 10.2 | 10.3 | 15.1 | 7.8 |
| | 24 Hours Later | 15.1 | 11.3 | 17.1 | 11.7 |
| | Target Value | 10 | 10 | 10 | 10 |

As shown in Table 4, the hair conditioning compositions of Test Examples 11 to 14 which contain a mono-long chain alkyl type quaternary ammonium salt or a combination of a tertiary amine and an organic acid forming the quaternary ammonium salt showed the result excellent in both hygroscopicity and water absorbability. In particular, the composition of Test Example 13 containing stearoxypropyltrimethylammonium chloride had remarkably high water absorbability.

Based on the above results, the hair conditioning compositions of the present invention can preferably contain a mono long-chain alkyl type quaternary ammonium salt as a cationic surfactant. In addition, it is also possible to act as the quaternary ammonium salt by containing a mono long-chain alkyl type tertiary amine and an organic acid.

<Use of Higher Fatty Acid>

The results of determination and evaluation of the samples using a higher fatty acid as the component (a) of the present invention (hair conditioning precursor compositions) are shown in the following Table 5. In Table 5, each determination and evaluation regarding an endothermic peak temperature, hygroscopicity, and water absorbability were carried out according to the methods described above.

Table 5

| | | Example 15 | Example 16 | Example 17 | Example 18 | Example 19 | Example 20 | Example 21 |
|---|---|---|---|---|---|---|---|---|
| Stearyl trimethyl ammonium chloride | | 13.9 | 13.9 | 13.9 | 13.9 | 13.9 | 13.9 | 13.9 |
| Palmitic acid | | 31.1 | 16.3 | 10.0 | 6.7 | 8.3 | 13.4 | 16.7 |
| Stearic acid | | | 16.3 | 23.4 | 15.6 | 19.5 | 31.2 | 39.0 |
| Erythritol | | 55.0 | 53.5 | 52.7 | 63.8 | 58.3 | 41.6 | 30.4 |
| Molar Ratio of Higher Fatty Acid (Compound) to Cationic Surfactant | | 3.0 | 3.0 | 3.0 | 2.0 | 2.5 | 4.0 | 5.0 |
| DSC Endothermic Peak Temperature [°C] | Gel | 85.4 | 82.4 | 83.9 | 86.4 | 85.3 | 82.8 | 82.2 |
| | Medium | 119.1 | 119.0 | 118.9 | 119.1 | 119.0 | 118.9 | 118.6 |
| | Evaluation | ○ | ○ | ○ | ○ | ○ | ○ | ○ |
| Hygroscopicity (Rate of Increased Weight after 6 Hours) | 90%RH | 36.0% | 41.0% | 40.0% | 38.5% | 37.0% | 35.0% | 33.5% |
| | 85%RH | 18.5% | 20.0% | 18.5% | 20.5% | 22.0% | 19.5% | 17.5% |
| | 75%RH | 2.0% | 3.0% | 4.0% | 1.5% | 3.5% | 4.0% | 3.0% |
| Water Absorbability (Dilution Rate after a Given Time) | 3 Hours Later | 3.7 | 4.6 | 4.5 | 4.2 | 4.5 | 4.6 | 4.8 |
| | 24 Hours Later | 5.5 | 6.0 | 5.9 | 4.9 | 5.8 | 6.2 | 6.2 |
| | Target Value | 5 | 5 | 5 | 5 | 5 | 5 | 5 |

As shown in Table 5, Test Examples 15 to 21 using a higher fatty acid alone or in combination as the component (a) of the present invention were hair conditioning compositions that had a preferable DSC endothermic peak temperature and also were excellent in water absorbability, as well as in the case of using a higher alcohol and/or derivative thereof.
In view of the above, it is obvious that, in the hair conditioning compositions of the present invention, a higher fatty acid can be preferably used as the component (a).

<Cationic Surfactant>

The determination and evaluation of the samples (hair conditioning precursor compositions) shown in Table 6 were carried out, and the cationic surfactant preferable as the component (b) of the present invention was studied. In Table 6, each determination and evaluation regarding an endothermic peak temperature, hygroscopicity, and water absorbability were carried out according to the methods described above. The results are shown in Table 6.

Table 6

| | | Example 22 | Example 23 | Example 24 |
|---|---|---|---|---|
| Stearyl trimethyl ammonium chloride (80% solution) | | 13.9 | | |
| Behenyl trimethyl ammonium chloride(80% solution) | | | 17.0 | |
| Stearamidopropyl dimethylamine | | | | 11.8 |
| Palmitic acid | | 10.0 | 10.0 | 10.0 |
| Stearic acid | | 23.4 | 23.4 | 23.4 |
| Erythritol | | 52.7 | 49.6 | 54.8 |
| Molar Ratio of Higher Fatty Acid (Compound) to Cationic Surfactant | | 3.0 | 3.0 | 3.0 |
| DSC Endothermic Peak Temperature [°C] | Gel | 83.9 | 87.3 | 60.6 |
| | Medium | 118.9 | 118.6 | 116.6 |
| | Evaluation | ○ | ○ | ○ |
| Hygroscopicity (Rate of Increased Weight after 6 Hours) | 90%RH | 40.0% | 40.0% | 35.5% |
| | 85%RH | 18.5% | 22.5% | 15.5% |
| | 75%RH | 4.0% | 3.5% | 2.5% |
| Water Absorbability (Dilution Rate after a Given Time) | 3 Hours Later | 4.5 | 4.3 | 1.4 |
| | 24 Hours Later | 5.9 | 5.3 | 1.2 |
| | Target Value | 5 | 5 | 5 |

As shown in Table 6, when a higher fatty acid was used as the component (a), a mono long-chain alkyl type quaternary ammonium salt (Test Examples 22 and 23) as (b) cationic surfactant was used, whereby a hair conditioning composition excellent as a hair conditioning precursor composition could be obtained.
In view of the above, also in the hair conditioning compositions of the present invention using a higher fatty acid as the component (a), a mono long-chain alkyl type quaternary ammonium salt can be preferably used as the component (b).
The formulation examples of the hair conditioning compositions of the present invention are shown below. However, the present invention is not limited to these formulation examples. The amount of the component contained is all shown in % by mass.

<Formulation Example 1 Hair treatment>

| | |
|---|---|
| Behenyl trimethyl ammonium chloride | 13.0 |
| Dicocoylethyl hydroxyethylmonium methosulfate | 1.0 |
| Cetyl alcohol | 12.0 |

(continued)

| | |
|---|---|
| Stearyl alcohol | 18.0 |
| Stearic acid | 0.3 |
| Erythritol | 26.20 |
| Glycerin | 5.0 |
| Dimethicone (500 cs) | 5.0 |
| Dimethiconol (1000 cs) | 5.0 |
| Methylphenyl methicone | 1.0 |
| PEG-10 dimethicone | 1.0 |
| 2-Octyldodecanol | 0.5 |
| Octyl palmitate | 3.0 |
| Mineral oil | 2.0 |
| Jojoba alcohol | 0.5 |
| Phenoxy ethanol | 3.0 |
| Perfume | 3.5 |
| (Production Method) | |

The mixture obtained by melting and stirring behenyl trimethyl ammonium chloride, dicocoylethyl hydroxyethylmonium methosulfate, cetyl alcohol, stearyl alcohol, and stearic acid under heating at 130°C and erythritol previously melted were mixed under heating until homogeneous. Furthermore, other components were added thereto, stirred and mixed, and the mixture was transferred to a container and cooled to room temperature, to give a composition.

The resulting composition was not sticky, was excellent in ease of handling, and could be preferably used as a hair conditioner by diluting with water by 6 times.

<Formulation Example 2 Hair conditioner>

| | |
|---|---|
| Stearyl trimethyl ammonium bromide | 8.0 |
| Stearamidopropyl trimethylammonium chloride | 5.0 |
| Distearyl dimethyl ammonium chloride | 1.0 |
| Stearyl alcohol | 23.0 |
| Behenyl alcohol | 8.0 |
| Isostearyl alcohol | 0.5 |
| Oleic acid monoglyceride | 0.3 |
| Maltitol | 26.1 |
| Isopentyldiol | 5.0 |
| Sorbitol | 4.0 |
| Dimethicone (100 cs) | 5.0 |
| (bis-isobutyl PEG-14/amodimethicone) copolymer | 0.5 |
| Isocetyl isostearate | 2.0 |
| Octyl palmitate | 3.0 |
| Di(phytosteryl/octyldodecyl) lauroyl glutamate | 1.0 |
| Camellia reticulata Lindl. seed oil | 1.0 |
| Alpha-tocopherol | 1.0 |
| Phytosteryl macadamiate | 1.0 |
| POE(1)-1,2-dodecanediol | 3.0 |
| Vanillyl butyl ether | 0.1 |
| Menthol | 1.0 |
| Perfume | 2.5 |
| (Production method) | |

The mixture obtained by melting and stirring stearyl trimethyl ammonium bromide, stearamidopropyl trimethylammonium chloride, distearyl dimethyl ammonium chloride, stearyl alcohol, behenyl alcohol, isostearyl alcohol, and oleic acid monoglyceride under heating at 140°C and maltitol, isopentyldiol, and sorbitol previously melted were mixed under heating until homogeneous. Furthermore, other components were added thereto, stirred and mixed, and the mixture was transferred to a container and cooled to room temperature, to give a composition.

The resulting composition was not sticky, was excellent in ease of handling, and could be preferably used as a hair conditioner by diluting with water by 8 times.

<Formulation Example 3 Hair conditioner>

| | |
|---|---|
| Cetyl trimethyl ammonium chloride | 5.0 |
| Behenyl PG trimethyl ammonium chloride | 5.0 |
| Stearamidopropyl dimethylamine | 2.0 |
| Cetyl alcohol | 20.0 |
| Stearyl alcohol | 20.0 |
| Isostearic acid | 0.5 |
| Polyethylene glycol (molecular weight 9000) | 25.0 |
| Dipropylene glycol | 5.0 |
| Lactic acid | 0.2 |
| Amodimethicone (1000 cs) | 5.0 |
| Lauryl polyglyceryl-3 polydimethylsiloxyethyl dimethicone | 0.5 |
| PEG/PPG-30/10 dimethicone | 0.5 |
| Octyl lactate | 2.0 |
| Isononyl isononate | 2.0 |
| Squalane | 0.2 |
| Purified sesame oil | 1.0 |
| Orange oil | 0.4 |
| Rosemary oil | 0.2 |
| Methyl paraben | 2.0 |
| Propyl paraben | 1.0 |
| Perfume | 2.5 |
| (Production method) | |

The mixture obtained by melting and stirring cetyl trimethyl ammonium chloride, behenyl PG trimethyl ammonium chloride, stearamidopropyl dimethylamine, lactic acid, cetyl alcohol, stearyl alcohol, and isostearic acid under heating at 85°C and polyethylene glycol (molecular weight of 9,000) and dipropylene glycol previously melted were mixed under heating until homogeneous. Furthermore, other components were added thereto, stirred and mixed, and the mixture was transferred to a container and cooled to room temperature, to give a composition.

The resulting composition was not sticky, was excellent in ease of handling, and could be preferably used as a hair conditioner by diluting with water by 10 times.

## Claims

**1.** A hair conditioning composition comprising:

(a) 10 to 90% by mass of one or more components selected from

(i) higher alcohols, being straight chain alcohols having 16 or more carbon atoms, and
(ii) higher fatty acids, having 12 to 22 carbon atoms, and
(iii) derivatives thereof, represented by following formulas (I) and (II)

$$R^1\text{-O-(-(CH}_2)_y\text{-O-)}_x\text{-H} \qquad \text{(I)}$$

wherein in the formula (I), $R^1$ is a straight chain or branched fatty alcohol residue having 10 to 24 carbon atoms, and each of x and y is an integer of 1 to 3,

$$R^2\text{-COO-CH}_2\text{-CH(OH)-CH}_2\text{-Q} \qquad \text{(II)}$$

wherein $R^2$ is a straight chain or branched fatty acid residue having 9 to 23 carbon atoms, and Q is H or OH;

(b) 5 to 35% by mass of a cationic surfactant, and
(c) one or more components selected from the group consisting of erythritol, maltitol, sorbitol, xylitol and/or polyethylene glycol with a molecular weight of 3,000 to 5,000,000, having a melting point of 155°C or less,

wherein the endothermic peak of a gel which is formed from (a) and (b) in the composition is 50°C or more as measured by a differential scanning calorimeter (DSC),
wherein the water content is 10% by mass or less, and
wherein the molar ratio of (a) to (b) is 2.5 or more to less than 6.0.

**2.** The hair conditioning composition according to claim 1, wherein in component (a) the straight chain alcohols have 16 to 22 carbon atoms and/or fatty acids, being straight chain fatty acids, have 16 to 22 carbon atoms.

**3.** The hair conditioning composition according to claim 1 or 2, wherein (b) is a

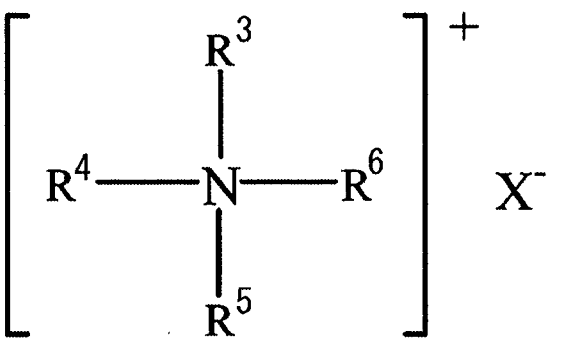

(III)

wherein $R^3$ represents a straight chain or branched alkyl group having 8 to 36 carbon atoms that may be substituted with a hydroxyl group, or

$$R^7\text{-(Z1)}_q\text{-(Y1)}_p\text{-},$$

$R^7$ represents a straight chain or branched alkyl group having 8 to 36 carbon atoms that may be substituted with a hydroxyl group, Y1 represents a linkage group selected from $-CH_2CH_2-$, $-CH_2CH_2CH_2-$, $-CH_2CH_2O-$, and $-CH(OH)CH_2O-$, and Z1 represents a linkage group selected from an amide bond (-CONH-), ether bond (-0·), and ester bond (-COO·), each of p and q represents an integer of 0 or 1,
$R^4$, $R^5$ and $R^6$ represent an alkyl group having 1 to 3 carbon atoms or benzyl group that may be substituted with a hydroxyl group and may be the same or different, X represents a halogen atom, an alkyl sulfate group having 1 or 2 carbon atoms, or anion that may form a salt with quaternary ammonium,

or the alkyl type quaternary ammonium salt is formed by a combination of the mono long-chain alkyl type amine represented by the following formula (IV) and an organic

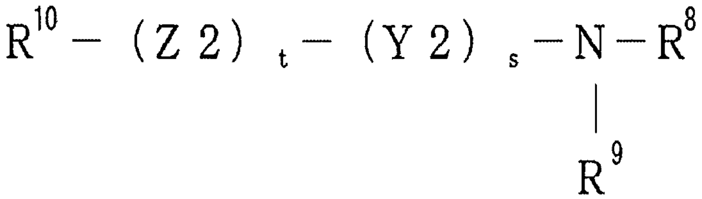

(IV)

$R^8$ and $R^9$ represent an alkyl group having 1 to 3 carbon atoms or benzyl group that may be substituted with a

hydroxyl group and may be the same or different;
$R^{10}$ represents a straight chain or branched alkyl group having 8 to 36 carbon atoms that may be substituted with a hydroxyl group;
Y2 represents a linkage group selected from $-CH_2CH_2-$, $-CH_2CH_2CH_2-$, $-CH_2CH_2O-$, and - $CH(OH)CH_2O-$, and Z2 represents a linkage group selected from an amide bond (-CONH-), ether bond (-0-), and ester bond (-COO-); each of s and t represents an integer of 0 or 1.

**4.** The hair conditioning composition according to claim 3, wherein the organic acid is a water soluble organic acid.

**5.** The hair conditioning composition according to any of claims 1 to 4, wherein component (c) comprises one or more components selected from the group consisting of erythritol, maltitol and/or polyethylene glycol with a molecular weight of 3,000 to 5,000,000.

**6.** The hair conditioning composition according to any of claims 1 to 5, wherein the polyethylene glycol has a molecular weight of 3,000 to 300,000.

**7.** The hair conditioning composition according to any of claims 1 to 6, being a solid or powder form at less than 50°C.

**8.** A method for producing a hair conditioner, comprising: mixing the hair conditioning composition of any of claims 1 to 7 with water.

**9.** A method of using the hair conditioning composition of any of claims 1 to 7, comprising: mixing the hair conditioning composition with water.

**10.** Method according to claim 9 comprising the dilution of the hair conditioning composition according to any of claims 1 to 7 with water at a dilution rate of 3 to 15 times by mass before use.

## Patentansprüche

**1.** Haarpflegezusammensetzung, enthaltend:

(a) 10 bis 90 Masse-% einer oder mehrerer Komponenten, die ausgewählt sind aus

(i) geradkettigen höheren Alkoholen mit 16 oder mehr Kohlenstoffatomen und
(ii) höheren Fettsäuren mit 12 bis 22 Kohlenstoffatomen und
(iii) deren Derivaten, die dargestellt werden durch die folgenden Formeln (I) und (II)

$$R^1\text{-O-(-(CH}_2)_y\text{-O-)}_x\text{-H} \qquad \text{(I)}$$

wobei $R^1$ in Formel (I) ein geradkettiger oder verzweigter Fettalkoholrest mit 10 bis 24 Kohlenstoffatomen ist und x und y jeweils eine ganze Zahl von 0 bis 3 sind,

$$R^2\text{-CCO-CH}_2\text{-CH(OH)-CH}_2\text{-Q} \qquad \text{(II)}$$

wobei $R^2$ ein geradkettiger oder verzweigter Fettsäurerest mit 9 bis 23 Kohlenstoffatomen ist und Q H oder OH ist;

(b) 5 bis 35 Masse-% eines kationischen Tensids und
(c) eine oder mehrere Komponenten, die gewählt sind aus der Gruppe bestehend aus Erythritol, Maltitol, Sorbitol, Xylitol und/oder Polyethylenglykol mit einem Molekulargewicht von 3.000 bis 5.000.000 und einem Schmelzpunkt von 155°C oder niedriger,

wobei das endotherme Maximum eines aus (a) und (b) gebildeten Gels in der Zusammensetzung 50°C oder höher beträgt, gemessen mit einem Differentialabtastkalorimeter (DSC), wobei der Wassergehalt 10 Masse-% oder weniger beträgt und wobei
das Molverhältnis von (a) zu (b) 2,5 oder höher bis weniger als 6,0 beträgt.

**2.** Haarpflegezusammensetzung gemäß Anspruch 1, wobei in Komponente (a) die geradkettigen Alkohole 16 bis 22 Kohlenstoffatome und/oder die Fettsäuren, die geradkettige Fettsäuren sind, 16 bis 22 Kohlenstoffatome enthalten.

**3.** Haarpflegezusammensetzung gemäß Anspruch 1 oder 2, wobei (b) ein quaternäres Ammoniumsalz des Mono-Langketten-Alkyltyps der Formel (III) ist

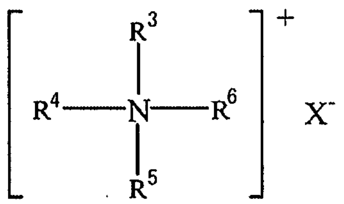

(III)

wobei $R^3$ eine geradkettige oder verzweigte Alkylgruppe mit 8 bis 36 Kohlenstoffatomen darstellt, die mit einer Hydroxylgruppe substituiert sein kann,oder

$R^7\text{-}(Z1)_q\text{-}(Y1)_p\text{-}$,

wobei $R^7$ eine geradkettige oder verzweigte Alkylgruppe mit 8 bis 36 Kohlenstoffatomen darstellt, die mit einer Hydroxylgruppe substituiert sein kann, Y1 eine Bindungsgruppe darstellt, die gewählt ist aus $-CH_2CH_2-$, $-CH_2CH_2-CH_2-$, $-CH_2CH_2-O-$ und $-CH(OH)CH_2O-$, und Z1 eine Bindungsgruppe darstellt, die aus einer Amidbindung (-CONH-), einer Etherbindung (-0-) und einer Esterbindung (-COO-) gewählt ist, wobei p und q jeweils eine ganze Zahl von 0 oder 1 sind,
wobei $R^4$, $R^5$ und $R^6$ eine Alkylgruppe mit 1 bis 3 Kohlenstoffatomen oder eine Benzylgruppe darstellen, die mit einer Hydroxylgruppe substituiert sein können und die gleich oder verschieden sein können, und wobei X ein Halogenatom, eine Alkylsulfatgruppe mit 1 oder 2 Kohlenstoffatomen oder ein Anion, das mit quaternärem Ammonium ein Salz bilden kann, darstellt
oder das quaternäre Ammoniumsalz des Alkyltyps gebildet wird durch eine Kombination des durch die folgende Formel (IV) dargestellten Amins des Mono-Langketten-Alkyltyps und einer organischen Säure,

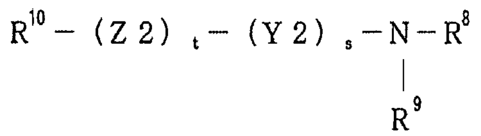

(IV)

wobei $R^8$ und $R^9$ eine Alkylgruppe mit 1 bis 3 Kohlenstoffatomen oder eine Benzylgruppe darstellen, die mit einer Hydroxylgruppe substituiert werden kann und die gleich oder verschieden sein kann;
wobei $R^{10}$ eine geradkettige oder verzweigte Alkylgruppe mit 8 bis 36 Kohlenstoffatomen darstellt, die mit einer Hydroxylgruppe substituiert sein kann,
wobei Y2 eine Bindungsgruppe darstellt, die gewählt ist aus $-CH_2CH_2-$, $-CH_2CH_2-CH_2-$, $-CH_2CH_2-O-$ und $-CH(OH)CH_2O-$, und wobei Z2 eine Bindungsgruppe darstellt, die aus einer Amidbindung (-CONH-), einer Etherbindung (-0-) und einer Esterbindung (-COO-) gewählt ist, wobei s und t jeweils eine ganze Zahl von 0 oder 1 darstellen.

**4.** Haarpflegezusammensetzung gemäß Anspruch 3, wobei die organische Säure eine wasserlösliche organische Säure ist.

**5.** Haarpflegezusammensetzung gemäß einem der Ansprüche 1 bis 4, wobei die Komponente (c) eine oder mehrere Komponenten enthält, die ausgewählt sind aus der Gruppe bestehend aus Erythritol, Maltitol und/oder Polyethylenglykol mit einem Molekulargewicht von 3.000 bis 5.000.000.

**6.** Haarpflegezusammensetzung gemäß einem der Ansprüche 1 bis 5, wobei das Polyethylenglykol ein Molekulargewicht von 3.000 bis 300.000 hat.

**7.** Haarpflegezusammensetzung gemäß einem der Ansprüche 1 bis 6, welche bei weniger als 50°C in Feststoff- oder Pulverform vorliegt.

**8.** Verfahren zum Herstellen einer Haarpflegezusammensetzung, umfassend: das Mischen der Haarpflegezusammensetzung gemäß einem der Ansprüche 1 bis 7 mit Wasser.

**9.** Verfahren zur Anwendung einer Haarpflegezusammensetzung gemäß einem der Ansprüche 1 bis 7, umfassend: das Mischen der Haarpflegezusammensetzung mit Wasser.

**10.** Verfahren gemäß Anspruch 9, umfassend das Verdünnen der Haarpflegezusammensetzung gemäß einem der Ansprüche 1 bis 7 mit Wasser bei einem Verdünnungsverhältnis des 3- bis 15-fachen der Masse vor Anwendung.

**Revendications**

**1.** Composition d'après-shampooing, comprenant:

(a) 10 à 90% en masse d'un ou plusieurs composants choisis parmi

(i) d'alcools supérieurs, ceux-ci étant des alcools à chaîne droite ayant 16 atomes de carbone ou plus, et
(ii) d'acides gras supérieurs ayant 12 à 22 atomes de carbone, et
(iii) de leur dérivés, représentés par les formules (I) et (II) suivantes

$$R^1\text{-O-(-(}CH_2)_y\text{-O-)}_x\text{-H} \qquad (I)$$

où $R^1$ dans la formule (I) est un résidu d'alcool gras à chaîne droite ou ramifié ayant 10 à 24 atomes de carbone, et x et y chacun étant un nombre entier compris entre 1 et 3,

$$R^2\text{-COO-}CH_2\text{-CH(OH)-}CH_2\text{-Q} \qquad (II)$$

où $R^2$ est un résidu de l'acide gras à chaîne droite ou ramifié ayant 9 à 23 atomes de carbone, et Q est H ou OH;

(b) 5 à 35% en masse d'un tensioactif cationique, et
(c) un ou plusieurs composants choisis dans le groupe consistant en érythritol, maltitol, sorbitol, xylitol et/ou polyéthylène glycol de poids moléculaire compris entre 3.000 et 5.000.000 ayant un point de fusion inférieur ou égal à 155°C,

le pic endothermique d'un gel formé de (a) et (b) dans la composition étant supérieur ou égal a 50°, mesuré par un calorimètre à balayage différentiel (DSC), la teneur en eau étant inférieure ou égale à 10% en masse et le rapport molaire de (a) à (b) étant égal ou supérieur à 2,5 à inférieur à 6,0.

**2.** Composition d'après-shampooing selon la revendication 1, dans laquelle les alcools à chaîne droite dans le composant (a) contiennent 16 à 22 atomes de carbone et/ou les acides gras, qui sont des acides gras à chaîne droite, contiennent 16 à 22 atomes de carbone.

**3.** Composition d'après-shampooing selon la revendication 1 ou 2, dans laquelle (b) représente un sel d'ammonium quaternaire de type mono-alkyle à chaîne longue de la formule (III)

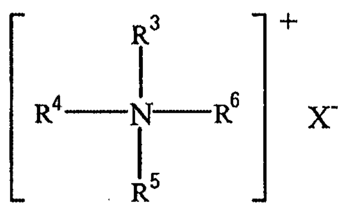

(III)

où $R^3$ représente un groupe alkyle à chaîne droite ayant 8 à 36 atomes de carbone pouvant être substitué avec un groupe hydroxyle, ou

$$R^7\text{-(Z1)}_q\text{-(Y1)}_p\text{-},$$

où $R^7$ représente un groupe alkyle à chaîne droite ou ramifié ayant 8 à 36 atomes de carbone pouvant être substitué avec un groupe hydroxyle, Y1 représente un groupe de liaison choisi parmi $-CH_2CH_2-$, $-CH_2CH_2-CH_2-$, $-CH_2CH_2-O-$ et $-CH(OH)CH_2O-$, et Z1 représente un groupe de liaison choisi parmi une liaison amide (-CONH-), une liaison éther (-0-) et une liaison ester (-COO-), chacun de p et q représentant un nombre entier de 0 ou 1, $R^4$, $R^5$ et $R^6$ représentent un groupe alkyle ayant 1 à 3 atomes de carbone ou un groupe benzyle pouvant être substitué avec un groupe hydroxyle et pouvant être identique ou différent, X représente un atome d'halogène, un groupe de sulfates d'alkyle ayant 1 ou 2 atomes de carbone, ou un anion qui peut former un sel avec l'ammonium quaternaire,
ou le sel d'ammonium quaternaire de type alkyle est formé par une combinaison de l'amine de type mono-alkyle á chaîne longue représenté par la formule (IV) suivante et d'un acide organique,

$$R^{10}-(Z2)_t-(Y2)_s-\underset{\displaystyle R^9}{\underset{|}{N}}-R^8 \qquad \text{(IV)}$$

où $R^8$ et $R^9$ représentent un groupe alkyle ayant 1 à 3 atomes de carbone ou un groupe benzyle pouvant être substitué avec un groupe hydroxyle et pouvant être identique ou différent;
$R^{10}$ représente un groupe alkyle à chaîne droite ou ramifié ayant 8 à 36 atomes de carbone pouvant être substitué avec un groupe hydroxyle,
Y2 représente un groupe de liaison choisi parmi $-CH_2CH_2-$, $-CH_2CH_2-CH_2-$, $-CH_2CH_2-O-$ et $-CH(OH)CH_2O-$, et Z2 représente un groupe de liaison choisi parmi une liaison amide (-CONH-), une liaison éther (-0-) et une liaison ester (-COO-), chacun de s et t représentant un nombre entier de 0 ou 1.

**4.** Composition d'après-shampooing selon la revendication 3, dans laquelle l'acide organique est un acide organique hydrosoluble.

**5.** Composition d'après-shampooing selon l'une des revendications 1 à 4, dans laquelle le composant (c) comprend un ou plusieurs composants choisis dans le groupe consistant en érythritol, maltitol et/ou polyéthylène glycol de poids moléculaire compris entre 3.000 et 5.000.000.

**6.** Composition d'après-shampooing selon l'une des revendications 1 à 5, dans laquelle le polyéthylène glycol a un poids moléculaire compris entre 3.000 et 300.000.

**7.** Composition d'après-shampooing selon l'une des revendications 1 à 6, ladite composition étant un produit solide ou poudreux à moins de 50°C.

**8.** Procédé de fabrication d'une composition d'après-shampooing, comprenant: mélanger la composition d'après-shampooing selon l'une des revendications 1 à 7 avec de l'eau.

**9.** Procédé d'utilisation de la composition d'après-shampooing, comprenant: mélanger la composition d'après-shampooing avec de l'eau.

**10.** Procédé selon la revendication 9, comprenant la dilution de la composition d'après-shampooing selon l'une des revendications 1 à 7 avec de l'eau à un taux de dilution de 3 à 15 fois en masse avant l'utilisation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008206157 A
- JP 2004534807 W
- JP 2005516026 W
- JP 2003300812 A
- WO 2006119042 A1